# EUROPEAN PATENT SPECIFICATION

(11) **EP 4 076 214 B1**
(45) Date of publication and mention of the grant of the patent: **15.01.2025**
(21) Application number: 20838926.2
(22) Date of filing: 15.12.2020
(51) Int. Cl.: A61B 17/02

(54) **TISSUE HOLDING DEVICE**
GEWEBEHALTEVORRICHTUNG
DISPOSITIF DE MAINTIEN DE TISSU

(30) Priority: 16.12.2019 CH 16252019
(43) Date of publication of application: 26.10.2022
(73) Proprietor: Solike Trading GmbH, 8915 Hausen am Albis ZH (CH)
(72) Inventor: GABI, Michael, 1400 Yverdon-les-Bains VD (CH); HEFERMEHL, Lukas, 8902 Urdorf ZH (CH)
(74) Representative: Rentsch Partner AG
(86) International application number: PCT/EP2020/086253
(87) International publication number: WO 2021/122618

(56) References cited:
- ES-T3- 2 547 723
- US-A1- 2010 185 048
- US-A1- 2010 274 161
- US-A1- 2014 121 464
- US-B1- 6 558 314

## Description

### FIELD OF THE INVENTION

The present invention relates to the field of laparoscope surgery, and in particular to tissue holding devices and tissue holding systems for moving and maneuvering a target tissue during such laparoscopic procedures.

### BACKGROUND OF THE INVENTION

Laparoscopy, also known as "minimal invasive surgery" or "keyhole surgery" has become a standard approach for the treatment of many tumors, in particular tumors within the abdominal or pelvic cavities. US2014/121464 A1, US2010/185048 A1, US6558314 B1, ES2547723 T3 and US2010/274161 A1 disclose tissue suction devices of the prior art

### SUMMARY OF THE INVENTION

Claim 1 defines the invention and dependent claims disclose embodiments. No surgical methods are claimed. Due to the vulnerable consistency of many organ tissues, tumor-grasping with conventional laparoscopic tools (e.g. laparoscopic forceps) can lead to tissue injuries during dissection, tumors spreading and bleeding, which may prolongate the operation time and impair organ function and oncologic outcomes.

For holding and maneuvering of the tissue with the tumor (in the following also referred to as target tissue), suction-based devices are known to which the tissue can be coupled via suction pressure. Corresponding devices are known, e. g. from US5196003A, JP2005253906A, and US20110230706A1. An integrated retrieval bag for retrieving resected material (e. g. tumorous tissue) is disclosed, e. g., in US6506166. Further, US20140121464A1 discloses a surgical vacuum device including a vacuum cup sized for attachment to and manipulation of a target tissue. The device further includes a vacuum hose for applying a vacuum to the interior of the cup, and structure for applying a tensioning force to the cup once it has been placed and a vacuum applied.

EP1800606A1 discloses a coronary artery bypass grafting device that includes a plurality of attachment members each having: a flexible tube; a suction cup portion provided in a tip end side of the flexible tube; a three-way cock provided in the flexible tube; and a holding member that holds the flexible tube. At least one of the attachment members further includes a duckbill valve provided in the flexible tube. The suction cup portion can be provided with a plurality of concave slits so that lateral sliding of the suction cup portion against tissues can be prevented.

It is an overall objective of the present invention to improve the state of the art of laparoscopic devices and methods, and in particular laparoscopic tissue holding devices and tissue holding systems and to overcome or reduce problems known in the art at least in part.

Favorably, a tissue holding device in accordance with the present disclosure is simple and cost-efficient in manufacture, and safe and convenient application. Favorably, it can be coupled to the tissue and moved and/or maneuvered with the tissue without the tissue being damaged for the before-explained reasons.

In an aspect, the overall objective is achieved by a laparoscopic tissue holding device. The tissue holding device incudes a rigid mouthpiece, the mouthpiece having an inner side and an outer side, wherein the inner side delimits a hollow inner space. The mouthpiece further has a proximal side and an opposed distal side, wherein the inner space is open at the distal side. The tissue holding device further includes a fluidic coupler arranged for fluidic coupling the inner space of the mouthpiece with a negative pressure supply.

The tissue holding device further includes a grasping member. The grasping member is arranged at the outer side of the mouthpiece and is designed for grasping the tissue holding device in particular with a medical forceps and/or pincers.

In this document, the expression " target tissue" refers to a tissue that is or shall be hold by the holding device. The target tissue may in particular be or include tissue to be resected, such as tumorous tissue. The directional expressions "distal" and "proximal" refer to directions pointing towards respectively away from the target tissue. The target tissue may for example be pathological tissue, such as tumor and/or cancer tissue. The expressions "fluid" respectively "fluidic" may generally refer to gases or gas mixtures, such as air as well as to liquids, such as water and/or saline solution. In dependence of the specific design and realization, the fluidic coupler may especially be a pneumatic coupler or a combined pneumatic/liquid coupler.

In a situation of use, the distal side of the mouthpiece faces the target issue, while the opposite proximal side points away from the target tissue, and the distal side of the mouthpiece contacts the target tissue. By applying a negative pressure respectively under-pressure in the inner space via the negative pressure supply, the target tissue is mechanically coupled to the mouthpiece via suction. The negative pressure causes the target tissue to be slightly sucked into the inner space. The distal side of the mouthpiece is generally formed by a distal-pointing circumferential front. The circumferential front may be flat or rounded and is designed to enable the establishment of a fluid-tight respectively sealing circumferential contact between the mouthpiece and the target tissue. A rounded front is preferable in order to avoid the target tissue to be damaged. Typically, the circumferential front lies in a plane, in particular in a plane traverse to a longitudinal axis of the mouthpiece. When viewed from the distal side (with a viewing direction from distal towards proximal), the mouthpiece is concave. The inner space accordingly forms a cavity that is open to the distal side.

By grasping the grasping member with a forceps or pincers while the target tissue is coupled to the mouthpiece via suction as explained before, the target tissue can be manipulated and in particular maneuvered or positioned in as safe manner without being damaged. Damage of the target tissue is to be avoided since a damage would result in the potential release of pathological cells, such as cancer cells, into the body. The grasping member is favorably rigidly coupled to the mouthpiece and may in an embodiment be formed integrally with the mouthpiece.

In an embodiment, the mouthpiece is rotational symmetric about a central longitudinal axis that extends between proximal and distal. In some of such embodiments, the mouthpiece is cup-shaped, dome-shaped or bell-shaped. For a rotationally symmetric mouthpiece and in particular a cup-shaped, dome-shaped or bell-shaped mouthpiece, the outer contour at the distal side (with a viewing direction from distal to proximal) is circular. In some embodiments, the mouthpiece, the grasping member and the fluidic coupler or a part thereof may, in combination, be rotationally symmetric about a common longitudinal axis. At the proximal side, the mouthpiece may have an apex or a flat or planar front surface. The longitudinal axis may run through the apex or a center of the front surface as well as through a center of the distal side of the mouthpiece.

In accordance with the claims, a surface of the inner side of the mouthpiece is structured. The structuring may be realized, e. g., by roughening the surface of the inner side of the mouthpiece, and/or by providing the surface of the inner side of the mouthpiece with protruding members, such as burls, ripples or splines that project into the inner space. In accordance with the claims, the structuring is realized by circumferentially arranged splines or ripples. In combination with the soft respectively elastic properties of the target tissue and the negative pressure, a structured surface as explained before creates a structural locking respectively positive locking between the target tissue and the mouthpiece, thereby preventing undesired relative motion or slipping between the mouthpiece and the target tissue. A surface is to referred as "structured" if it significantly deviates from a smooth surface.

In an embodiment, an outer surface of the grasping member is structured. Providing the grasping member with a structured outer surface serves the purpose of preventing undesired relative motion between the laparoscopic tissue holding device respectively the mouthpiece and the forceps or pliers. The structuring may favorably realize by providing the outer surface of the grasping member for example with a roughed surface, and/or providing members, such as burls and/or a knurling in further variants, the structuring is realized by splines, such as circumferential and/or longitudinal splines. The structuring of the grasping member ensures that the forces and/or torques for maneuvering respectively positioning the target tissue via the mouthpiece can be safely and reliably transferred from the forceps or pliers to the mouthpiece.

In an embodiment, the grasping member has a cylindrical shape and in particular a cylindrical circumferential surface that may be structured as explained before. Favorably, the grasping member is arranged directly at the outer side of the mouthpiece and projects outwards from the mouthpiece respectively in proximal direction.

The fluidic coupler is arranged at the outside of the mouthpiece. In some embodiments, the fluidic coupler includes a generally tubular fitting with a through-going fluidic lumen, with one end of the fluidic channel opening into the inner space of the mouthpiece. The fitting is favorably shaped to be received in an end section of a flexible tubing respectively for putting the end section a flexible tubing over the fitting, such that the flexible tubing circumferences the fluidic fitting in a fluid-tight manner.

In some embodiments, the fluidic coupler includes a flexible tubing. The tubing has a first tubing side and an opposed second tubing side, wherein the first tubing side is configured for fluidic coupling to the negative pressure supply, and wherein the second tubing side is fluidic coupled with the inner space. The coupling of the second tubing side with the with the inner space may be realized via a fitting as mentioned before. The first tubing side is generally designed or prepared for fluidic coupling with the negative pressure supply, being directly or via intermediate components such as a valve and/or a liquid trap. The tubing has a continuous respectively through-going lumen that extends between the first and second tubing side.

The second tubing side is favorably mechanically secured to the mouthpiece and/or the fitting, e. g. by welding, gluing, and/or clamping. In some embodiments, the coupling between fitting and tubing may be releasable.

In some favorable embodiment, the fluidic coupler has a diameter of no more than 3.5mm respectively is designed for connection with a tube of no more than 2 mm inner diameter for establishing the fluidic coupling. Favorably, the flexible tubing has an outer diameter of no more than 3mm. Small diameters for the fluidic coupler and the tubing are generally preferable. The reason is that during application the tube passes through the trocar and it is favorable if the tubing only occupies as little of the available space of the trocar as possible, thereby allowing the same trocar to be used for further instruments respectively devices.

In further embodiments, the flexible tubing is not part of the fluidic coupler but a flexible tubing as explained before is attached to the fluidic coupler directly prior to application.

In an embodiment, the grasping member is arranged around the fluidic coupler as a sleeve. An annular space may be formed between an inner side of the grasping member and the fluidic coupler, with the annular space receiving an end section, in particular an end section at the second side of the tubing. The fluidic coupler and the grasping member may in particular be arranged coaxially. For this type of embodiment, the grasping member serves simultaneously as protection member that prevents the tubing from being potentially damaged by the pliers or forceps.

In an embodiment, the grasping member includes a fluidic passage, the fluidic passage fluidically connecting the inner space of the mouthpiece and the fluidic coupler. In such embodiment, the grasping member may be directly attached to the mouthpiece at its outer side, and the fluidic coupler may be directly attached to the grasping member at its opposite side, resulting in the grasping member being arranged between the mouthpiece and the fluidic coupler. The fluidic passage of the grasping member is realized as through-going lumen. This type of design is particularly favorable regarding manufacture, in particular via injection molding and is particularly compact respectively space-saving. Further, an interference between the tubing and further instruments during the surgical procedure is minimized or fully avoided.

In an embodiment, at least a portion of the fluidic coupler and/or the grasping member are arranged coaxially around a longitudinal axis, in particular a central axis of the mouthpiece. In embodiments where the fluidic coupler includes a fitting as explained before, the fitting may be arranged coaxially around the axis. In embodiments where the fluidic coupler includes the tubing, an end section at the second side of the tubing may be arranged coaxially around the axis.

In an embodiment, the mouthpiece is dimensioned to pass through a trocar, the trocar having an inner diameter of no more than 30mm, in particular no more than 14mm.

In an embodiment, a maximal lateral extension of the mouthpiece traverse to a longitudinal axis, in particular a central axis of the mouthpiece, does not exceed 75mm and does preferably not exceed 50mm or 20mm. In embodiments where the mouthpiece is rotationally symmetric and for example or dome-shaped, cup-shaped or bell-shaped as explained before, the maximal lateral extension may correspond to a diameter of the mouthpiece at the distal side. Generally, in typical embodiments, the lateral dimension of the mouthpiece is maximum at the distal side and narrows towards the proximal side.

In an embodiment, the mouthpiece is transparent. A transparent mouthpiece is in particular favorable to allow visual inspection of the inner volume during application, e. g. using an endoscope.

In some embodiments, the grasping member is formed integrally with the mouthpiece. Further in some embodiments, the fluidic coupler is fully or partly formed integrally with the mouthpiece. In particular, a fitting as explained before may be formed integrally with the mouthpiece.

The mouthpiece may be made from any suited medical grade polymeric material, for example a thermoplastic polymer, by way of injection molding, if applicable together with the grasping member and/or the fluidic coupler respectively a fitting as explained before. In alternative embodiments, the tissue holding device and in particular the mouthpiece is made from metal, for example stainless steel or titanium. Typically, the tissue holding device is designed for single use and is discarded afterwards, but may in principle also designed for multiple use. In some embodiments, the Young's modulus of the mouthpiece is in a range of 1MPa to 1000GPa.

In a further aspect, the overall objective is achieved by a laparoscopictissue holding system. The tissue holding system includes a laparoscopic tissue holding device according to any embodiment as explained before and/or further below. The tissue holding system further includes a negative pressure supply.

In embodiments where the tissue holding device does not directly include a flexible tubing, the tissue holding system further includes a flexible tubing for fluidic coupling the inner space of the mouthpiece with the negative pressure supply.

In some embodiments, the tissue holding system further includes a liquid trap as generally known in the art. The liquid trap prevents any liquid, such as body liquid and/or liquid originating from a liquid supply as explained below, to be suck into the negative pressure supply.

In an embodiment, the laparoscopic tissue holding system further includes a positive pressure supply and/or a liquid supply and includes a switching valve. The fluidic coupler is alternatively fluidic coupleable with the negative pressure supply and the positive pressure supply or liquid supply, respectively via the switching valve.

In such embodiment, the first tubing side is generally coupled with the one port of the switching valve, while the negative pressure supply and the positive pressure supply/liquid supply are coupled to further posts of the switching valve, thereby allowing coupling of the first tubing side alternatively with the negative pressure supply of positive pressure supply/liquid supply.

In some embodiments, the switching valve has a further intermediate state where the first tubing side is fluidic isolated and neither coupled with the negative pressure supply nor the positive pressure supply/liquid supply.

Providing a positive pressure supply and/or liquid supply in addition to the negative pressure supply is favorable to allow a flushing in case of blood clots or particles being present in the tubing, fluidic coupler and/or inner space, without having to withdraw the tissue holding device out of the body and without interrupting the surgical procedure. Further, it allows to safely relieve the tissue from the tissue holding device, in particular the mouthpiece, by generating an overpressure rather than a suction pressure/under pressure within the inner volume of the mouthpiece.

It is to be understood that the term "positive pressure supply" may in general refer to both a positive pressure gas supply and/or a positive pressure liquid supply. A positive pressure supply may accordingly be designed as gas supply to provide any suited and favorably substantially inherent gas, such as medical-grade air. A liquid supply may be designed to supply any suited liquid, in particular saline solution.

The positive pressure supply or liquid supply may, for example, be realized as a syringe as known in the art, with the syringe being filled with gas or liquid. Such syringe may be manually operated or via a syringe driver as known in the art. Other positive pressure supplies, such as a compressor and/or a pump. Further, the overpressure supply may be provided as part of the readily installed infrastructure, e. g. in a hospital OR.

Similarly, the negative pressure supply may, for example, be provided as suction pump respectively vacuum pump, or as part of the infrastructure.

In a further aspect, the overall objective is achieved by the use of a laparoscopictissue holding device and/or a laparoscopic tissue holding system according to any embodiment as described above and/or further below in a laparoscopic surgical procedure. In still further aspect, the overall objective is achieved by a laparoscopic surgical method respectively surgical procedure using a laparoscopic tissue holding device and/or the laparoscopic tissue holding system is disclosed.

In an embodiment, the procedure includes introducing the tissue holding device respectively its mouthpiece into a body cavity via a previously placed trocar, such that the tubing extends through the trocar to the outside of a patient's body.

Further in an embodiment, the procedure includes the step of mechanically coupling a target tissue inside the body cavity, in particular a pathological target tissue like a tumorous tissue, to the mouthpiece by generating a negative pressure respectively suction pressure within the inner space of the mouthpiece via a negative pressure supply in fluidic coupling with the inner space of the mouthpiece. The negative pressure supply may be activated prior to or after positioning the mouthpiece relative to the target tissue.

Further in an embodiment, the procedure includes the step of grasping the gasping member of the holding device with a forceps or pliers that is introduced into the body cavity via the same or a further trocar.

Further in an embodiment, the procedure includes manipulating, in particular positioning and/or maneuvering, the mouthpiece together with the target tissue by moving the forceps or pliers.

Further in an embodiment, the procedure includes cutting out respectively resecting the target tissue while being coupled to the mouthpiece. The cutting is made by a surgical device, such as laparoscopic scissors or a cauterization device, as generally known in the art. Favorably, the cut is made into healthy tissue spaced some distance apart from the pathological and/or tumorous tissue. The target tissue accordingly includes the pathological tissue, surrounded by non-pathological respectively healthy tissue. The surgical device may be introduced into the body cavity by the same trocar as the tissue holding device or a further trocar.

Further in an embodiment, the procedure includes the step of withdrawing the holding device respective its mouthpiece via the trocar, with the target tissue being coupled to the mouthpiece, respectively with the negative pressure supply still being active.

In an alternative embodiment, the tissue holding device is not withdrawn directly from the body cavity with the target tissue being coupled. Instead, the procedure may include the step of introducing a retrieval bag as generally known in the art via the same trocar as the tissue holding device or a further trocar. In such embodiment, the procedure includes the step of maneuvering the mouthpiece with the target tissue via the forceps or pliers to a retrieval bag that has been previously introduced and positioned inside the body cavity. The retrieval bag may be introduced via the same trocar as the tissue holding device or a further trocar. Subsequently, the procedure may include the step of releasing the target tissue from the mouthpiece, thereby placing the target tissue inside the retrieval bag. Such release may be performed by deactivating respectively switching off the negative pressure supply. Optionally, the releasing may further include the step of fluidically coupling the inner space of the mouthpiece with a positive gas pressure supply or liquid supply as explained before, thereby, actively forcing the target tissue away from the mouthpiece and into the retrieval back. Finally, such embodiment may include the step of separately withdrawing the tissue holding device and the retrieval bag with the target tissue from the body cavity.

In a variant, the procedure includes, subsequent to maneuvering the mouthpiece with the target tissue to the retrieval back, cutting the tubing close to the mouthpiece, e. g. using scissors that are introduced into the body cavity, and placing the mouthpiece together with the target tissue in the retrieval bag. In this variant, the procedure includes the step of retrieving the retrieval bag together with the mouthpiece and the target tissue positioned therein. Subsequently, the retrieval bag is closed, thereby encapsulating its content, and retrieved.

Optionally, the procedure may further include the step of temporarily releasing the target tissue from the mouthpiece by coupling the inner space of the mouthpiece with a positive pressure supply and/or liquid supply, and subsequently coupling the target tissue again with the mouthpiece by coupling the inner space of the mouthpiece with the negative pressure supply. Such steps may be applied for removing blood clots or particles during the procedure as required.

### BRIEF DESCRIPTION OF THE DRAWINGS

- Fig. 1: shows an embodiment of a laparoscopic tissue holding system in a schematic functional view;
- Fig. 2a: shows an embodiment of a laparoscopic tissue holding device in a side view;
- Fig. 2b: shows the laparoscopic tissue holding device of Fig. 2a in a perspective view;
- Fig. 3: partly shows a further embodiment of a laparoscopictissue holding device in a schematic side view;
- Fig. 4a: schematically shows a step of laparoscopic surgical procedure;
- Fig. 4b: schematically shows a further step of a laparoscopic surgical procedure;
- Fig. 4c: schematically shows a still further step of a laparoscopic surgical procedure;
- Fig. 4d: schematically shows a still further step of a laparoscopic surgical procedure;
- Fig. 4e: schematically shows a variant of the step as shown in Fig. 4d.

### Exemplary embodiments

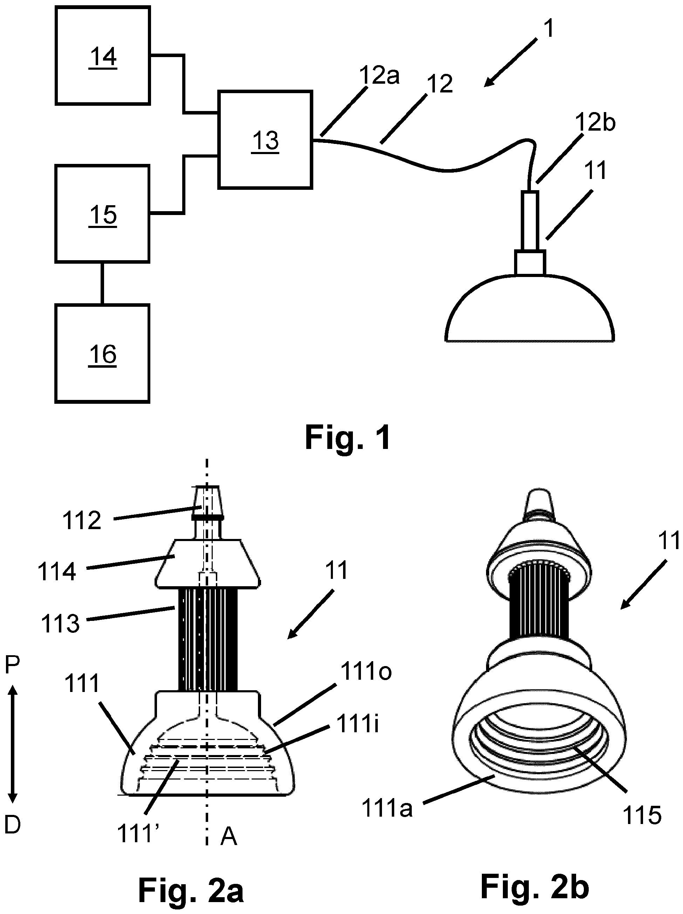

In the following, reference is first made to Figure 1. Figure 1 shows an exemplary embodiment of a laparoscopic tissue holding system 1 in accordance with the present invention in a schematic functional view. The tissue holding system 1 includes tissue holding device 11, flexible tubing 12, optional switching valve 13, optional positive pressure supply 14, optional liquid trap 15, and negative pressure supply 16.

Exemplary designs for the tissue holding device 11 are discussed in more detail further below. The flexible tubing 12 is at its second tubing side 1 2b connected to the tissue holding device 11 as explained further below. Alternatively, the flexible tubing 12 may be formed integrally with the tissue holding device 11 and be a part thereof.

An opposed end respectively first tubing side 12a is connected to a switching valve 13. Via the switching valve 13, the first tubing side 12a can be fluidically coupled with a negative pressure supply 16 via a liquid trap 15, or alternatively to a positive pressure supply 14. In another embodiment, the first tubing side 1 2a is coupled with the negative pressure supply 16 via the liquid trap 15, with the switching valve 13 and the positive pressure supply 14 being omitted. The switching valve 13 is typically manually operated but may also be automatically operated via an actuator.

In the following, reference is additionally made to Figure 2a and Figure 2b, showing an exemplary embodiment of the tissue holding device 11 in a side view and perspective view, respectively. The tissue holding device 11 includes a rigid mouthpiece 111, a fluidic coupler 112, a grasping member 113 and in this design a hub 114, all of which are exemplary formed integrally as a common and substantially rigid plastic component which is favorably optically transparent.

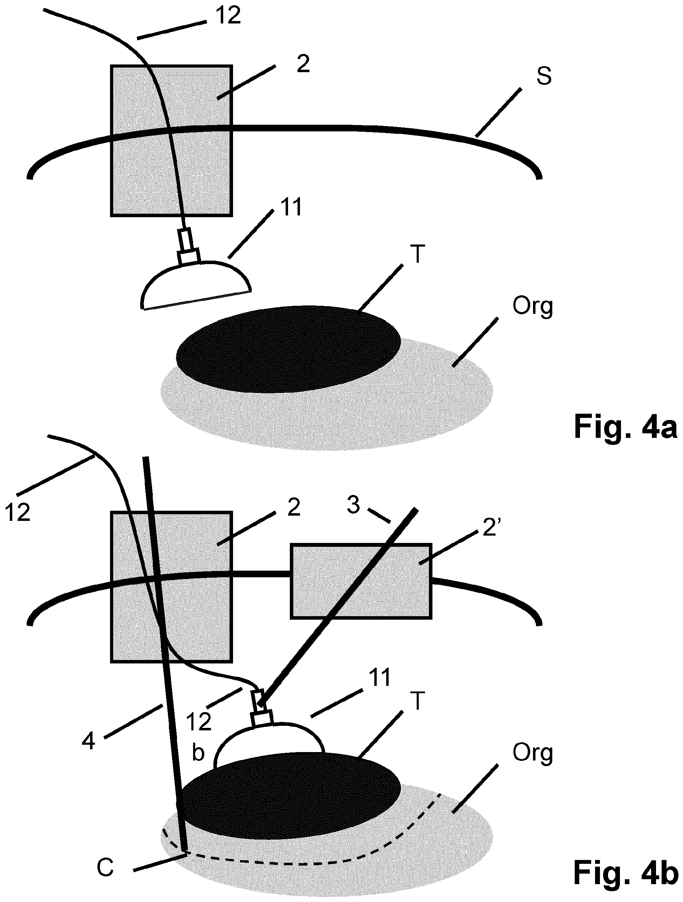

The mouthpiece 111 is in this embodiment generally bell-shaped respectively cup-shaped and has an outer side 111o and an inner side 111i, the latter delimiting a hollow inner space 111' that is open at a distal side of the mouthpiece. The proximal and distal direction, respectively, are indicated as arrows "P", "D". At the distal side, the mouthpiece has an in this example planar front 111a. For application, a negative pressure respectively suction pressure is established in the inner space 111', with the circumferential front 111a establishing a fluid-tight seal together with the soft target tissue T (see Figure 4a - 4d), thereby coupling the mouthpiece and the target tissue. Due to the suction pressure, the target tissue is generally slightly suck into the inn space 111' to ensure a safe coupling during manipulation. The inner side 111i is structured, exemplary by a number of circumferential splines 115 that protrude into the inner space 11 1'. The tissue holding device 11 is rotationally symmetric around a longitudinal axis A that extends between proximal and distal.

At its proximal side, the mouthpiece 111 has in this example a flat top surface (not referenced) from which the grasping member 113 extends in proximal direction. The grasping member 113 has in this embodiment a generally cylindrical respectively tubular shape and a structured outer surface which is realized by a plurality of circumferentially distributed longitudinal splines. The structure of the grasping member 113 ensures safe gripping when maneuvering respectively manipulating the mouthpiece 111 with a forceps or pliers.

At the proximal end of the grasping member 113, an in this example frustoconical hub 114 is arranged as intermediate element. From the proximal end of the hub, the fluidic coupler 112 in form of a fitting extends in proximal direction. Priorto application or already during manufacture, an end section at the second tubing side 12b is placed around the fitting 112, such that the fitting 112 is located inside the end section of the tubing 1 2, and the tubing 12 abuts the proximal front face of the hub 114. The tubing 12 may be permanently fixed to the fluidic coupler 112 e. g. during manufacture as explained before. Alternatively, the end section of the tubing 12 at the second tubing side 1 2b may be clamped onto the fitting 112 from the outside, and/or may have a somewhat smaller inner diameter than an outer diameter of the fitting 11 2 and be coupled by way of flexible radial expansion. A continuous and axially through-going fluidic passage is present in the fluidic coupler 112, the hub 114 and the grasping member 113, thereby fluidic coupling the inner volume or lumen of the tubing 12 with the inner space 111'.

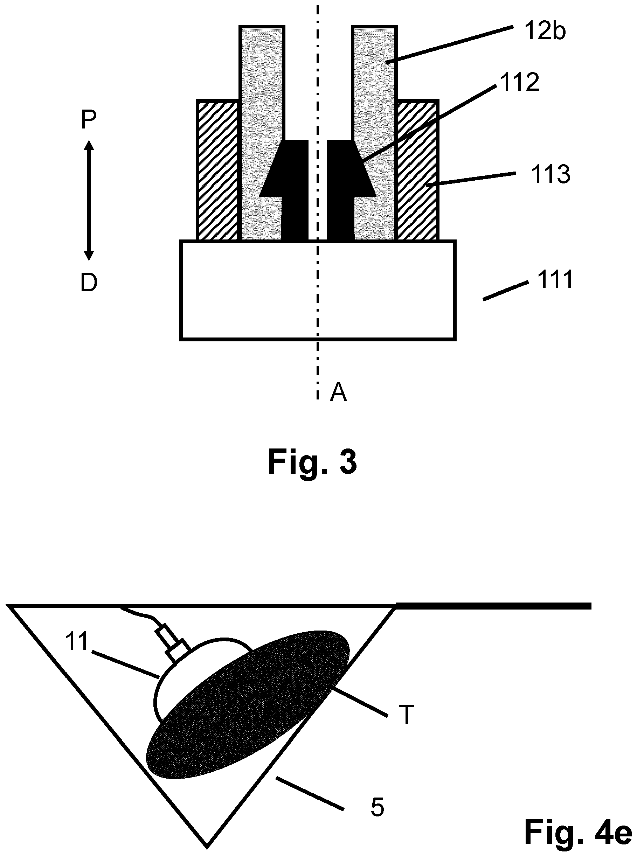

In the following, reference is additionally made to Figure 3. Figure 3, shows a proximal portion of the tissue holding device 11 in an alternative embodiment. In the design of Figure 3, the fluidic coupler respectively fitting 11 2 directly projects from an exemplarily cylindrical proximal end portion of the mouthpiece 111 in proximal direction. In this embodiment, the grasping member 113 is shaped as a tubular sleeve and arranged coaxially around the fitting 11 2. Like the fluid coupler 11 2, the grasping member 113 extends from the proximal front surface of mouthpiece 111 in proximal direction. The end section at the second tubing side 12b is arranged in an annular space between the grasping member 1 13 and the fitting 112.

It is noted that, while referred to as dedicated element, the tubing 12 may also be considered as fluidic coupler together with the fitting 11 2.

In the following, reference is additionally made to Figure 4a to Figure 4e, illustrating various steps and variants of a laparoscopic surgical procedure using a tissue holding device 11 and tissue holding system 1.

**Figure 4a** schematically shows an organ Org that is located in a body cavity of a patient's body (not shown as such), with S indicating the patient's skin.

The organ Org has a tumorous tissue respectively tumor T that shall be resected and removed by way of laparoscopy. The tumor T further defines a target tissue as explained before in the general description. Via a trocar 2 as generally known in the art, the tissue holding device 11 is introduced into the body cavity together with a portion at the second tubing side 1 2b. The tubing 12 extends through the trocar 2 to the outside of the patient's body.

**Figure 4b** illustrates a subsequent step where the mouthpiece 111 of the tissue holding device 11 is coupled to the tumor T by way of suction as explained before. The negative pressure may be generated by activating the negative pressure supply 16 (not shown in Fig. 4b) prior to or after maneuvering the tissue holding device 11 to its desired position at the tumor T. Maneuvering of the tissue holding device 11 is done with forceps 3 that is introduced into the body cavity in this example through a further trocar 2'. The tumor T is resected respectively cut of the tissue of organ Org using laparoscopic scissors 4 which is in this example also introduced into the body cavity via the trocar 2. A cut C (indicated as dashed line) is made in the healthy tissue of the organ Org in some distance of the tumor T to ensure that the tissue of the tumor T is not damaged. During the cutting, the tumor T and the surrounding tissue are maneuvered and positioned as desired using the forceps 3 and the tissue holding device 11.

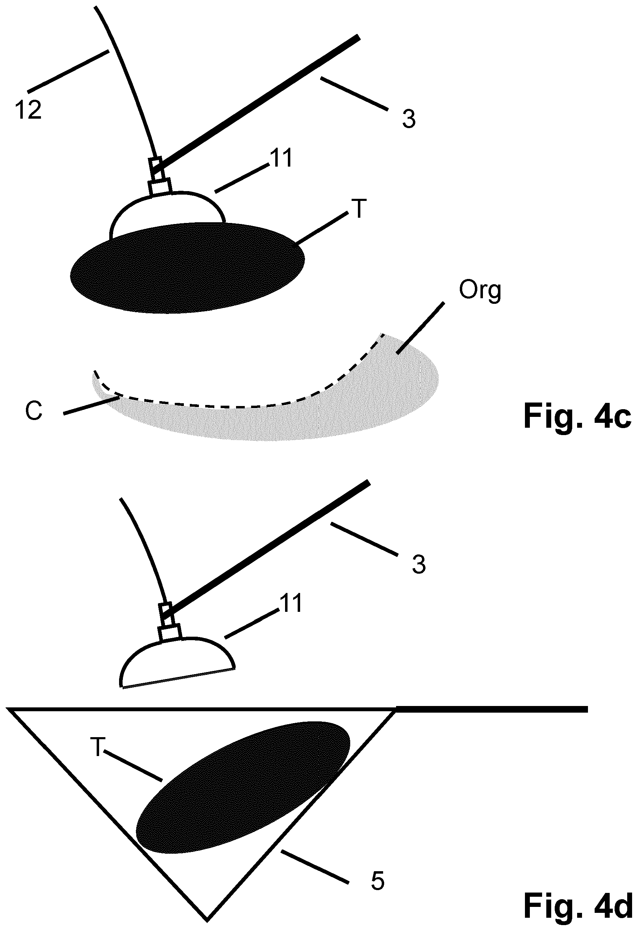

After finishing the resection, the tissue holding device 11 with the tumor T (and some surrounding tissue) is moved away from the remaining tissue of the organ Org, as schematically illustrated in **Figure 4c****.**

By way of the forceps 3, the tissue holding device 11 with the tumor T is maneuvered to a retrieval bag 5 that is also placed in the body cavity via a trocar. Finally, the tumor T is decoupled from the tissue holding device 11 and released into the retrieval bag 5. In dependence of the design of the tissue holding system 1, this may be done by simply switching off or deactivating the negative pressure supply 16. Alternatively, the tumor T may be actively forced away from the tumor T by activating the positive pressure supply 14 as explained before. **Figure 4d** schematically illustrates the situation where the tumor T has been released into the retrieval bag 5. Finally, the retrieval bag 5 with the tumor T and all further devices and instruments, in particular the tissue holding device 11 and the forceps 3, are withdrawn through the corresponding trocar or trocars out of the body cavity.

In a variant that is schematically illustrated in **Figure 4e****,** the tissue holding device 11 is maneuvered into the retrieval bag 5 together with the tumor T, and the tubing 12 is subsequently cut close to the tissue holding device 11 respectively it's fluidic coupler 112. In this variant, the tissue holding device 11 remains in the retrieval bag 5 together with the tumor 5 and is discarded therewith.

### REFERENCE SIGNS

- 1: Laparoscopic tissue holding system
- 11: Laparoscopic tissue holding device
- 111: mouthpiece
- 111': inner space
- 111a: circumferential front
- 111i: inner side
- 111o: outer side
- 112: fluidic coupler / fitting
- 113: grasping member
- 114: hub
- 115: protruding members / splines
- 12: tubing
- 12a: first tubing side
- 12b: second tubing side
- 13: switching valve
- 14: positive pressure supply
- 15: liquid trap
- 16: negative pressure supply
- 2,2': trocar
- 3: forceps
- 4: scissor
- 5: retrieval bag
- C: cut
- Org: organ
- S: skin
- T: tumor/ target tissue
- A: axis
- D: distal direction
- P: proximal direction

## Claims

1. Laparoscopic tissue holding device (11), the laparoscopic tissue holding device (11), including:
a) a rigid mouthpiece (111), the mouthpiece having an inner side (111i) and an outer side (111o), wherein the inner side (111i) delimits a hollow inner space (111'), the mouthpiece further having a proximal side and an opposed distal side, wherein the inner space (11 1') is open at the distal side;
b) a fluidic coupler (112) arranged for fluidic coupling the inner space (11 1') of the mouthpiece (111) with a negative pressure supply (16);
c) a grasping member (113), the grasping member (113) being arranged at the outer side (111o) of the mouthpiece (111) and being designed for grasping the laparoscopic tissue holding device (11) in particular with a medical forceps and/or pincers,
wherein a surface of the inner side (111i) of the mouthpiece (111) is structured, **characterized in that** the structuring is realized by a number of circumferential splines (115) or ripples that project into the inner space (11 1').

2. Laparoscopic tissue holding device (11) according to claim 1, wherein the mouthpiece (111) is rotational symmetric about a central longitudinal axis (A) that extends between proximal and distal.

3. Laparoscopic tissue holding device (11) according to either of the preceding claims, wherein an outer surface of the grasping member (11 3) is structured.

4. Laparoscopic tissue holding device (11) according to either of the preceding claims, wherein the grasping member (11 3) is arranged around the fluidic coupler (11 2) as a sleeve.

5. Laparoscopic tissue holding device (11) according to either of claims 1 to 4, wherein the grasping member (113) includes a fluidic passage, the fluidic passage fluidically connecting the inner space (11 1') of the mouthpiece (111) and the fluidic coupler (112).

6. Laparoscopic tissue holding device (11) according to either of the preceding claims, wherein at least a portion of the fluidic coupler (112) and/or the grasping member (113) are arranged coaxially around a longitudinal axis (A), in particular a central axis of the mouthpiece (111).

7. Laparoscopic tissue holding device (11) according to either of the preceding claims, wherein the mouthpiece (111) is dimensioned to pass through a trocar (2), the trocar (2) having an inner diameter of no more than 30mm, in particular no more than 14mm.

8. Laparoscopic tissue holding device (11) according to either of the preceding claims, wherein a maximal lateral extension of the mouthpiece (111) traverse to a longitudinal axis (A), in particular a central axis of the mouthpiece (111), does not exceed 75mm and does preferably not exceed 50mm or 20mm.

9. Laparoscopic tissue holding device (11) according to either of the preceding claims, wherein the mouthpiece (111) is transparent.

10. Laparoscopic tissue holding device (11) according to either of the preceding claims, wherein the fluidic coupler (112) includes a flexible tubing (12), the flexible tubing (12) having a first tubing side (12a) and an opposed second tubing side, wherein the first tubing side (1 2a) is configured for fluidic coupling to the negative pressure supply (16), and wherein the second tubing side (12b) is fluidic coupled with the inner space (11 1').

11. Laparoscopic tissue holding system (1), the Laparoscopic tissue holding system (1) including:
a) a laparoscopic tissue holding device (11) according to either of the preceding claims;
b) a negative pressure supply (16).

12. Laparoscopic tissue holding system (1) according to claim 11, wherein the laparoscopic tissue holding system (1) further includes a positive pressure supply and/or a liquid supply (14) and includes a switching valve (13), wherein the first tubing side (1 2a) is alternatively fluidic coupleable with the negative pressure supply (16) and the positive pressure supply or liquid supply, respectively via the switching valve (13).

## Patentansprüche

1. Laparoskopische Gewebehaltevorrichtung (11), wobei die laparoskopische Gewebehaltevorrichtung (11) umfasst:
a) ein starres Mundstück (111), wobei das Mundstück eine Innenseite (111i) und eine Außenseite (111o) aufweist, wobei die Innenseite (111i) einen hohlen Innenraum (11 1') begrenzt, wobei das Mundstück ferner eine proximale Seite und eine gegenüberliegende distale Seite aufweist, wobei der Innenraum (111') an der distalen Seite offen ist;
b) einen Fluidikkoppler (112), der zur Fluidikkopplung des Innenraums (11 1') des Mundstücks (111) mit einer Unterdruckversorgung (16) angeordnet ist;
c) ein Greifelement (113), wobei das Greifelement (113) an der Außenseite (111o) des Mundstücks (111) angeordnet ist und zum Greifen der laparoskopischen Gewebehaltevorrichtung (11) insbesondere mit einer medizinischen Pinzette und/oder Zange ausgebildet ist,
wobei eine Oberfläche der Innenseite (111i) des Mundstücks (111) strukturiert ist, **dadurch gekennzeichnet, dass** die Strukturierung durch eine Anzahl von in den Innenraum (11 1') hineinragenden umlaufende Rillen (115) oder Riffelungen realisiert ist.

2. Laparoskopische Gewebehaltevorrichtung (11) nach Anspruch 1, wobei das Mundstück (111) rotationssymmetrisch um eine zentrale Längsachse (A) ist, die sich zwischen proximal und distal erstreckt.

3. Laparoskopische Gewebehaltevorrichtung (11) nach einem der vorhergehenden Ansprüche, wobei eine äußere Oberfläche des Greifelements (113) strukturiert ist.

4. Laparoskopische Gewebehaltevorrichtung (11) nach einem der vorhergehenden Ansprüche, wobei das Greifelement (113) als Hülse um den Fluidikkoppler (112) angeordnet ist.

5. Laparoskopische Gewebehaltevorrichtung (11) nach einem der Ansprüche 1 bis 4, wobei das Greifelement (113) einen Fluidikkanal aufweist, der den Innenraum (11 1') des Mundstücks (111) und den Fluidikkoppler (112) fluidisch verbindet.

6. Laparoskopische Gewebehaltevorrichtung (11) nach einem der vorhergehenden Ansprüche, wobei zumindest ein Abschnitt des Fluidikkopplers (112) und/oder des Greifelements (113) koaxial um eine Längsachse (A), insbesondere eine Mittelachse des Mundstücks (111), angeordnet ist.

7. Laparoskopische Gewebehaltevorrichtung (11) nach einem der vorhergehenden Ansprüche, wobei das Mundstück (111) so dimensioniert ist, dass es durch einen Trokar (2) hindurchgeht, wobei der Trokar (2) einen Innendurchmesser von nicht mehr als 30 mm, insbesondere nicht mehr als 14 mm aufweist.

8. Laparoskopische Gewebehaltevorrichtung (11) nach einem der vorhergehenden Ansprüche, wobei eine maximale seitliche Ausdehnung des Mundstücks (111) quer zu einer Längsachse (A), insbesondere einer Mittelachse des Mundstücks (111), 75 mm nicht überschreitet und vorzugsweise 50 mm oder 20 mm nicht überschreitet.

9. Laparoskopische Gewebehaltevorrichtung (11) nach einem der vorhergehenden Ansprüche, wobei das Mundstück (111) transparent ist.

10. Laparoskopische Gewebehaltevorrichtung (11) nach einem der vorhergehenden Ansprüche, wobei der Fluidikkoppler (112) einen flexiblen Schlauch (12) umfasst, wobei der flexible Schlauch (12) eine erste Schlauchseite (12a) und eine gegenüberliegende zweite Schlauchseite aufweist, wobei die erste Schlauchseite (12a) für eine Fluidkupplung mit der Unterdruckversorgung (16) konfiguriert ist, und wobei die zweite Schlauchseite (12b) fluidisch mit dem Innenraum (11 1') gekoppelt ist.

11. Laparoskopisches Gewebehaltesystem (1), wobei das laparoskopische Gewebehaltesystem (1) umfasst:
a) eine laparoskopische Gewebehaltevorrichtung (11) nach einem der vorhergehenden Ansprüche;
b) eine Unterdruckversorgung (16).

12. Laparoskopisches Gewebehaltesystem (1) nach Anspruch 11, wobei das laparoskopische Gewebehaltesystem (1) ferner eine Überdruckversorgung und/oder eine Flüssigkeitsversorgung (14) und ein Schaltventil (13) aufweist, wobei die erste Schlauchseite (1 2a) über das Schaltventil (13) wahlweise mit der Unterdruckversorgung (16) und der Überdruckversorgung bzw. Flüssigkeitsversorgung fluidisch koppelbar ist.

## Revendications

1. Dispositif laparoscopique de maintien des tissus (11), le dispositif laparoscopique de maintien des tissus (11), comprenant :
a) une embouchure rigide (111), l'embouchure ayant une face interne (111i) et une face externe (111o), la face interne (111i) délimitant un espace intérieur creux (111'), l'embouchure ayant en outre une face proximale et une face distale opposée, l'espace intérieur (111') étant ouvert au niveau de la face distale ;
b) un coupleur fluidique (112) agencé pour coupler fluidiquement l'espace intérieur (11 1') d' embouchure (111) avec une alimentation en pression négative (16) ;
c) un élément de préhension (113), l'élément de préhension (113) étant disposé sur le côté extérieur (111o) de l'embouchure (111) et étant conçu pour saisir le dispositif laparoscopique de maintien des tissus (11), en particulier avec un forceps et/ou une pince médicale,
dans lequel une surface de la face interne (111i) d'embouchure (111) est structurée, **caractérisée par le fait que** la structuration est réalisée par un certain nombre de cannelures circonférentielles (115) ou d'ondulations qui font saillie dans l'espace intérieur (1 1 1').

2. Dispositif laparoscopique de maintien des tissus (11) selon la revendication 1, dans lequel l'embouchure (111) présente une symétrie de rotation autour d'un axe longitudinal central (A) qui s'étend entre le proximal et le distal.

3. Dispositif laparoscopique de maintien des tissus (11) selon l'une ou l'autre des revendications précédentes, dans lequel une surface extérieure de l'élément de préhension (113) est structurée.

4. Dispositif laparoscopique de maintien des tissus (11) selon l'une ou l'autre des revendications précédentes, dans lequel l'élément de préhension (113) est disposé autour du coupleur fluidique (112) sous la forme d'un manchon.

5. Dispositif laparoscopique de maintien des tissus (11) selon l'une ou l'autre des revendications 1 à 4, dans lequel l'élément de préhension (113) comprend un passage fluidique, le passage fluidique reliant fluidiquement l'espace intérieur (11 1') de l'embouchure (111) et le coupleurfluidique (112).

6. Dispositif laparoscopique de maintien des tissus (11) selon l'une ou l'autre des revendications précédentes, dans lequel au moins une partie du coupleur fluidique (112) et/ou de l'élément de préhension (113) sont disposés coaxialement autour d'un axe longitudinal (A), en particulier un axe central de l'embouchure (111).

7. Dispositif laparoscopique de maintien des tissus (11) selon l'une ou l'autre des revendications précédentes, dans lequel l'embouchure (111) est dimensionné pour passer à travers un trocart (2), le trocart (2) ayant un diamètre intérieur ne dépassant pas 30 mm, en particulier ne dépassant pas 14 mm.

8. Dispositif laparoscopique de maintien des tissus (11) selon l'une ou l'autre des revendications précédentes, dans lequel une extension latérale maximale de l'embouchure (111) par rapport à un axe longitudinal (A), en particulier un axe central de l'embout (111), ne dépasse pas 75 mm et ne dépasse de préférence pas 50 mm ou 20 mm.

9. Dispositif laparoscopique de maintien des tissus (11) selon l'une ou l'autre des revendications précédentes, dans lequel l'embouchure (111) est transparent.

10. Dispositif laparoscopique de maintien des tissus (11) selon l'une ou l'autre des revendications précédentes, dans lequel le coupleurfluidique (112) comprend une tubulure flexible (12), la tubulure flexible (12) ayant un premier côté de tubulure (12a) et un deuxième côté de tubulure opposé, dans lequel le premier côté de tubulure (1 2a) est configuré pour un couplage fluidique à l'alimentation en pression négative (16), et dans lequel le deuxième côté de tubulure (12b) est couplé fluidiquement à l'espace intérieur (111').

11. Système laparoscopique de maintien des tissus (1), le système laparoscopique de maintien des tissus (1) comprenant :
a) un dispositif laparoscopique de maintien des tissus (11) selon l'une ou l'autre des revendications précédentes ;
b) une alimentation en pression négative (16).

12. Système laparoscopique de maintien des tissus (1) selon la revendication 11, dans lequel le système laparoscopique de maintien des tissus (1) comprend en outre une alimentation en pression positive et/ou une alimentation en liquide (14) et comprend une soupape de commutation (13), dans laquelle le premier côté de tubulure (12a) est alternativement couplable fluidiquement avec l'alimentation en pression négative (16) et l'alimentation en pression positive ou l'alimentation en liquide, respectivement par l'intermédiaire de la soupape de commutation (13).
